Europäisches Patentamt

European Patent Office

Office européen des brevets

(11) Publication number:

**0 273 754**
**A2**

## (12) EUROPEAN PATENT APPLICATION

(21) Application number: **87311479.7**

(51) Int. Cl.⁴: **A 61 K 49/00**

(22) Date of filing: **29.12.87**

(30) Priority: **29.12.86 US 946950**

(43) Date of publication of application:
**06.07.88 Bulletin 88/27**

(84) Designated Contracting States:
**CH DE FR GB IT LI**

(71) Applicant: **CITY OF HOPE NATIONAL MEDICAL CENTER**
**1500 East Duarte Road**
**Duarte California 91010 (US)**

(72) Inventor: **Beatty, John David**
**1532 Hyland Avenue**
**Arcadia, California 91006 (US)**

**Beatty, Barbara Gwendolyn**
**1532 Hyland Avenue**
**Arcadia, California 91006 (US)**

**Duda, Rosemary Bernadette**
**3200 North Lane Shore Drive**
**Chicago, Illinois 60657 (US)**

(74) Representative: **De Minvielle-Devaux, Ian Benedict Peter**
**et al**
**CARPMAELS & RANSFORD 43, Bloomsbury Square**
**London WC1A 2RA (GB)**

(54) Method of enhancing the biodistribution of antibody for localization in lesions.

(57) A method for enhancing the biodistribution of antibody for localization in lesions comprising initially exposing a mammalian subject containing lesion-associated marker substance to antibody specific for the marker substance, and after a period of time such that the marker substance has associated with the antibody, reexposing the mammal to a further amount of the antibody.

EP 0 273 754 A2

Bundesdruckerei Berlin

**Description**

METHOD OF ENHANCING THE BIODISTRIBUTION OF ANTIBODY FOR LOCALIZATION IN LESIONS

BACKGROUND OF THE INVENTION

This invention relates to methods of enhancing the biodistribution in a mammalian subject. More specifically this invention relates to the use of monoclonal antibodies for localization, detection, and treatment of lesions, including tumors, in humans.

The diagnostic and therepeutic use of radioactively labeled antibodies specific to substances produced by or associated with tumors to detect or locate tumors is recognized. U.S. Patent No. 3,927,193 to Hansen et al. describes a method for determining the site of tumors which produce or are associated with carcinoembryonic antigen (CEA) using labeled antibodies to CEA. U.S. Patent No. 4,311,688 to Burchiel et al. describes the detection of cells that produce human chorionic gonadotropin (hCG) and compounds similar to the beta-chain of hCG by first administering anti-hCG or anti-hCG-beta and then administering radioactively labeled antibodies to anti-hCG-beta or anti-hCG. U.S. Patent No. 4,331,647 to Goldenberg describes both the detection of tumors using mixtures of radiolabeled antibody fragments specific to tumor-associated markers and tumor radiotherapy using antibody fragments labeled with radiotherapeutically effective radioisotopes. U.S. Patent No. 4,478,815 to Burchiel et al. likewise describes the use of radio-labeled antibody fragments to detect tumors producing hCG or other tumor associated antigen.

A major problem encountered in the use of radiolabeled antibodies to detect or treat tumors is the accumulation of radioactivity caused by radiolabeled antibodies, antibody fragments, or their metabolites in the blood pool, in interstitial fluids, or other tissues such as the liver and spleen. Such accumulation of radioactivity in these other areas of the body significantly reduces the resolution of tumor localization using radiolabeled antibodies. The detection or localization of lesions located in or near the liver is particularly difficult because radiolabeled substances tend to accumulate naturally in the liver.

In an effort to solve the problem of accumulated radioactivity in the body, U.S. Patent No, 4,348,376 to Goldenberg, describes the localization of tumors associated with CEA by concurrently administering both a radiolabeled antibody specific to CEA, and a background compensating material, normal immunoglobulin from the same or different species as that used to prepare the antibody, which is radiolabeled with a different radioisotope of the same element used to label the antibody to CEA. The level of radioactivity of the labeled normal immunoglobulin is used to determine the distribution of background radioactivity due to non-targeted specific antibody, which background distribution is then subtracted from the total activity. See also U.S. Patent No. 4,444,744 to Goldenberg which similarly describes a method for detecting other tumor-associated antigens.

U.S. Patent No. 4,460,561 to Goldenberg describes a method of tumor therapy wherein thermal neutrons excite a boron-10 isotope-containing antibody which has been localized by detection of an attached radioisotope label.

A further method of diminishing nontumor-associated antibody is described in Goldenberg, PCT Patent No. W08500522. A radiolabeled tumor-specific antibody is injected into the subject, and at a time after injection of the antibody sufficient to permit maximum selective uptake thereof by the tumor, a second, non-radiolabeled antibody specific against the first radiolabeled antibody is injected. The second antibody binds with an amount of labeled antibody not associated with tumor tissue such that the level of circulating (nontumor-associated) radiolabeled antibody is decreased.

There remains a need for a method for enhancing the biodistribution of antibody which requires neither the use of more than one type of antibody nor more than one type of label.

In the following description of the invention, the term "biodistribution" refers to the distribution of antibody in a subject to which antibody has been administered. By constrast, "uptake" refers to the quantity of antibody in a given tissue. The terms "lesion-associated marker substance", "marker substance", or "marker" refers to a substance produced by or associated with a lesion and for which an administered antibody is specific. A lesion is an abnormal change in the structure of an organ or part due to injury or disease, e.g., a tumor.

SUMMARY OF THE INVENTION

In general, the invention features a method for enhancing the biodistribution of antibody for localization in lesions comprising initially exposing a mammalian subject containing a lesion-associated marker substrate to antibody specific for the marker substance, and after a period of time such that the marker substance has associated with the antibody, reexposing the mammal to a further amount of the antibody.

In one preferred embodiment, the subsequently administered antibody is labeled with an agent detectable by radioimaging, photoimaging, or other suitable means. Imaging of lesion-associated marker is enhanced due to reduced uptake of the subsequently administered antibody by liver and spleen tissue.

In the other preferred embodiment, the subsequently administered antibody is labeled with a therapeutically effective agent. The therapeutic effects of this agent with respect to the lesion are enhanced due to reduced uptake of the therapeutic-labeled antibody by non-lesion tissue.

The methods of this invention are also advantageous because only one type of antibody and one type of label is needed for enhancing the biodistribution.

Other features and advantages of the invention will be apparent from the following description of the preferred embodiments, and from the claims.

## DETAILED DESCRIPTION

A method has been discovered for enhancing the biodistribution of antibody to a lesion-associated marker in a mammalian subject. Initially, a mammalian subject containing a marker substance produced by or associated with a lesion is exposed to an antibody. At a time after the initial exposure such that the subject recognizes, identifies, or associates with the antibody, the subject is again exposed to the same antibody. Exposure to the antibody may be accomplished by, for example, intravenous, parenteral or subcutaneous injection or oral administration. The first exposure of the subject to the antibody causes the subject to recognise the antibody which results in an enhanced biodistribution of that antibody upon the subject's subsequent reexposure to it. Specifically, the first exposure to a given antibody causes a decrease in the uptake of the subsequently administered labeled or unlabeled antibody by the liver and spleen. This allows more of the subsequently administered antibody to remain in the blood and eventually reach the lesion situs.

The decreased uptake of antibody by the liver and spleen which is produced by the methods of this invention is useful in a variety of contexts, for example, lesion detection and location in humans. In particular, lesions situated in or near the human liver and spleen are more easily detected and located due to the reduced uptake of labeled antibody by those tissues.

A human subject in which the presence and/or location of a lesion is to be ascertained by imaging means is first exposed to an antibody specific for a given lesion-associated marker. After a number or duration of exposures, and after a period of time such that the subject has come to recognize the antibody, the subject is exposed to the same antibody which is labeled with a detectable agent. This label could be a radionuclide such as indium-111 or technecium-99m suitable for gamma imaging, or could be a magnetic contrast agent such as gadolinium, manganese, or iron for magnetic resonance imaging. By subsequently photoscanning or employing other suitable methods for detecting the site or sites of label accumulation, the presence and/or location of lesions may be determined.

Application of the methods of this invention is also useful for providing a highly specific lesion therapy. A human subject having a lesion with which is associated a marker substance is first exposed to an antibody specific for that marker. After a period of time such that the subject has come to recognize that antibody, the subject is exposed to the same antibody which is labeled with a therapeutically effective agent such as yttrium-90 or methotrexate. The recognition induced in the subject by prior exposure to the antibody leads to reduced association of the labeled antibody with non-lesion tissue and concomitantly, increased association with lesion-associated marker substance. Thus, the destructive effects of the therapeutically effective agent against lesion cells are enhanced.

The following example illustrates the enhanced biodistribution of a given antibody caused by prior exposure of the subjects to the same antibody to which they are subsequently exposed. Specifically, it illustrates the phenomenon of recognition by which specific organs in a subject, exposed to a given antibody, by some means identifies or recognizes this antibody upon a later exposure to it. The uptake of this antibody is greately reduced due to the identification of the antibody by those organs.

### Example

Nude mice were injected with 5 x $10^6$ LS174T cells subcutaneously on day zero. Tumors were palpable on day seven.

The mice were divided into five groups. To the first group, a control, phosphate buffered saline (PBS) was administered. Different monoclonal antibody pretreatment regimes were administered to the other four groups of mice. For the initial exposure step (or pretreatment) PBS or one of the three types of monoclonal antibody (MAB) were used. All three MABs were specific for carcinoembryonic antigen (CEA).

The mice were administered either the PBS control or monoclonal antibodies intraperitoneally on days 7, 8, 9 and 10. The total MAB pretreatment dose of 2.5 mg was given as 1.0, 0.5, 0.5 and 0.5 mg on those successive days. The MAB pretreatment dose of 0.25 mg was given at one-tenth these doses on those successive days. All five groups of mice were injected with 2.5 micrograms T84.66 MAB labeled with 5 micro curies of indium-111 on day 13. On day 15, randomly selected animals from each group were imaged by photoscintigraphy. On day 16, the biodistributions of labeled T84.66 were determined.

One of the monoclonal antibodies used for pretreatment, T84.66, is the same, except for the absence of a label, as that to which all five groups of mice were subsequently exposed in the second step. The other two types of antibodies, ZCE 025 and T84.12, differ from T84.66. ZCE 025 is similar to T84.66 in that it is an $IgG_1$ (immunoglobulin G), but differs in that it is specific for a different CEA epitope than is T84.66. T84.12 differs from T84.66 in that it is an $IgG_{2a}$ rather than an $IgG_1$. T84.12 is, however, specific for the same epitope of CEA as is T84.66.

In the following table "n" denotes the number of mice in each of the five groups.

## TABLE 1

### MURINE BIODISTRIBUTION OF LS174T SUBCUTANEOUS XENOGRAPHS—THE EFFECT OF ANTIBODY EXPOSURE PRIOR TO ADMINISTRATION OF INDIUM-111 LABELED T84.66 (MEAN ± SE)

| Pre-treatment Substance | Phosphate Buffered Saline | ZCE 025 | T84.12 | T84.66 | T84.66 |
|---|---|---|---|---|---|
| Pre-treatment Dose (mg) | -- | 2.5 mg | 2.5 mg | 2.5 mg | 0.25 mg |
| n | 10 | 9 | 8 | 9 | 6 |
| Indium Labeled T84.66 | 2.5 ug | 2.5 ug | 2.5 ug | 2.5 ug | 2.5 ug |

**(a)  % 10/g ± SE***

| | | | | | |
|---|---|---|---|---|---|
| Blood(B) | 5.95±1.79 | 4.03±1.29 | 2.27±0.75 | 13.57±1.17 | 7.39±1.08 |
| Liver(L) | 24.69±4.43 | 24.31±2.81 | 29.63±2.77 | 8.35±1.21 | 5.83±0.43 |
| Spleen | 16.88±2.93 | 9.40±1.40 | 14.62±0.88 | 7.25±0.70 | 6.28±0.52 |
| Kidney | 11.96±4.31 | 8.40±0.79 | 6.46±0.65 | 12.81±1.26 | 11.51±0.83 |
| Lung | 3.81±1.02 | 3.04±1.08 | 1.83±0.40 | 8.16±1.07 | 5.58±0.82 |
| Tumor(T) | 38.67±10.49 | 24.15±7.18 | 17.65±3.96 | 10.89±0.68 | 16.42±1.47 |

**(b)  %ID/organ ± SE****

| | | | | | |
|---|---|---|---|---|---|
| Blood | 9.63±2.97 | 6.21±1.95 | 3.55±1.21 | 20.90±1.91 | 16.35±2.20 |
| Liver | 23.71±3.73 | 24.01±2.51 | 29.70±2.22 | 8.23±1.34 | 8.40±0.86 |
| Tumor | 8.80±1.46 | 15.95±1.42 | 11.44±1.85 | 6.99±1.71 | 9.68±2.38 |

**(c)  Ratios ± SE*****

| | | | | | |
|---|---|---|---|---|---|
| (T/L) | 3.59±1.43 | 1.56±0.69 | 0.72±0.21 | 1.42±0.11 | 2.90±0.33 |
| (T/B) | 8.20±1.04 | 6.36±0.45 | 9.72±1.30 | 0.82±0.32 | 2.59±0.57 |
| (L/B) | 14.53±4.33 | 12.75±3.03 | 25.96±6.52 | 0.63±0.09 | 1.05±0.39 |

**(d)  Tumor Wt (g) ± SE**

| | | | | | |
|---|---|---|---|---|---|
| | 0.57±0.17 | 1.14±0.23 | 0.82±0.13 | 0.78±0.2 | 0.48±0.13 |

---

\*      %ID/g, percent injected dose per gram of tissues.
\*\*     %ID/organ, percent injected dose per organ.
\*\*\*    ratio of %ID/g for two different tissues.

The first column of Table 1 shows that the PBS control followed by administration of indium-labeled T84.66 resulted in the greatest uptake in tumor and liver (%ID/g). In keeping with this, the scintiscan image had shown localization of intensity primarily in the tumor and the liver. The ratios of uptake (%ID/g) shown in Table 1 for the PBS control are 3.6 and 8.2 for tumor to liver and tumor to blood, respectively. The liver to blood ratio was very high at 14.5. The last column of Table 1 shows that prior administration of 0.25 mg of T84.66 followed by administration of indium-labeled T84.66 resulted in the greatest uptake in tumor alone (%ID/g). In keeping with this, the scintiscan images had shown localization of intensity in the tumor alone. The ratios of uptake (%ID/g) shown in Table 1 for the 0.25 mg T84.66 pretreatment were 2.9 and 2.6 for tumor to liver and tumor to blood, respectively. However, the liver to blood ratio was very low at 1.1.

The higher dose of T84.66 used for initial exposure (2.5 mg) resulted in lower liver uptake, but also lower uptake in the tumor. This resulted in all tissue ratios being near unity (1). This data shows that prior administration of T84.66 results not only in decreased accumulation of labeled T84.66 antibody in the liver, but also in increased retention of labeled T84.66 in the blood. Such retention in the blood is consistent with decreased accumulation in the liver. Moreover, such accumulation in the blood is consistent with increased tumor uptake, since, in contrast to antibody taken up by the liver, antibody in the blood may eventually become associated with the marker substance for which it is specific.

The example also shows that prior administration of an antibody similar, but not identical, to the subsequently administered antibody does not cause an enhanced biodistribution of the subsequently administered antibody. Specifically, as shown in the second column of Table 1, prior administration of ZCE 025 antibody, which differs from T84.66 in its CEA epitope specificity, yielded a high liver upate (24.3% injected dose per gram) and a high liver to blood ratio (12.8). This liver uptake and liver to blood ratio are far higher than those caused by prior administration of the T84.66 antibody at both the high (2.5 mg) and the low (0.25 mg) doses (liver uptake 8.4 and 5.8% ID/g, and L/B ratios 0.6 and 1.1 respectively). Similarly, prior administration of T84.12 antibody, which shares T84.66's epitope specificity but is an $IgG_{2a}$ rather than an $IgG_1$ antibody, yielded a high liver uptake (29.6% ID/g) and a very high liver to blood ratio (26.0). Thus, despite their similarity to T84.66, prior administration of both T84.12 and ZCE 025 did not enhance the biodistribution of subsequently administered T84.66.

Though the example illustrates the enhanced biodistribution of a particular antibody to a particular marker substance or antigen, the instant invention is not to be construed to be limited to the exemplified antibody-antigen pair.

In summary, the example shows that prior exposure to the same antibody as is subsequently administered has two effects. First, the uptake of subsequently administered antibody by the liver and spleen is significantly reduced. Second, more of the subsequently administered antibody is retained in the blood and is thus available for localization to the lesion-associated marker substance.

The period of time between the initial exposure to antibody and the subsequent exposure to antibody which may be labeled with an imaging or therapeutic substance, i.e., the time necessary to allow the subject to identify or recognize the antibody, is preferably from about one week to about three weeks. The duration of antibody administration for the initial exposure is preferably a period of about four days.

The antibodies used in the method of this invention may be specific for intracellular, cytoplasmic, or cell surface marker substances. Fragments of antibodies may also be used. These antibodies are preferably monoclonal of mammalian origin and may be synthesized by genetic engineering technology well known in the art, or they may be synthesized in vitro or in vivo utilizing genetic engineering technology.

Marker substances may be produced by lesions, or they may simply accumulate in, on, or near lesions. Substances suitable as markers for which antibodies used in accordance with this invention may be specific generally include, but are not limited to oncofetal antigens, tumor antigens, receptors, hormones, and enzymes. Examples of such marker substances are human chorionic gonadotropin (HCG) and/or its beta subunit, alpha-fetoprotein (AFP), Ca 19-9, CA125, 132CS, tissue polypeptide antigen, calon-specific antigen-p ($CSA_p$), A 431, TA-4, prostatic acid phosphatase, pancreatic oncofetal antigen, T101, S 100, parathormane, B1.1, B72.3, placental alkaline phosphatase, calcitonin, mammary tumor-associated glycoproteins, galactyasyl transferase-II (gt-II), glial fibrillary acidic protein (GFA), tumor angiogenesis factor (TAF), prostatic acid phosphatase, pregnancy $beta_1$-globulin, $beta_2$-microglobulin ovarian cystadenocarcinoma-associated antigen (OCAA), ovarian tumor-specific antigen, common glioma antigen (CGA), cervical cancer antigens, glioembryonic antigen (GEA), common meningioma antigen (CMA), terminal deoxynucleotidyl transferase (TAT), and cytoplasmic melanoma-associated antigens.

Substances which may be used as detectable labels for the antibodies used in the methods of this invention include various isotopes of the transition metals. In general, any transition metal isotope that gives off alpha particles, beta particles, gamma rays, or any other emission that could be used for detection or therapy may be used. These include but are not limited to iodine-131, iodine-123, iodine-126, iodine-133, bromine-77, fluorine-18, indium-111, indium 113m, technetium-99m, gallium-67, gallium-68, ruthenium-95, ruthenium-97, ruthenium-103, ruthenium-105, mercury-197, mercury-203, scandium-47, tellurium-121m, tellurium-122m, tellurium-125m, rhenium-99m, rhenium-101, rhenium-105, thulium-165, thulium-167, thulium-168, thallium-202, and antimony. Magnetic contrast agents such as gadolinium, manganese, and iron, which do not emit particles or rays, possess a property which is identifiable by the agents' ability to resonate at a certain frequency due to magnetic resonance, and as such may also be used as detectable labels.

Substances which may be used as therapeutic labels for the antibodies used in the methods of this invention include various isotopes of the transition metals and chemotherapeutic drugs. Thse include, but are not limited to yttrium-90, methotrexate, copper-67, indium-194, cobalt, carbon-14, daunomycin, vincristine, 5-fluorouracil, BCNU, nitrogen mustard, vinblastin, cyclophosphamide, actinomycin D, 6-mercaptopurine, arabinosyl, cytosine, L-asparaginase, adriamycin, cytoxin, busulfan, chlorambucil, thiotepa, melphalan, thioguanine, hydroxyurea, procarbazine, o,p-D,D,D, FUDR, mithramycin, bleomycin, DTIC, CCNU, and cis-platinum.

In addition to a therapeutically effective label, a radioactive or otherwise detectable level may also be attached to or otherwise associated with the subsequently administered antibody.

In another application of the methods of this invention, a human subject is first exposed to a given marker-specific antibody. Subsequently, the same antibody, now labeled with a detectable label and boron-10, is administered to the subject. The lesion is located by detecting the site or sites of labeled subsequently administered antibody accumulation. The lesion with which boron-10-labeled antibody is now associated is later irradiated with a well collimated beam of thermal neutrons. Such irradition gives rise to cytotoxic particles which have a therapeutic effect. The prior exposure of the human subject to an antibody the same as is subsequently administered diminishes uptake of said subsequently administered antibody by the liver and spleen, and thus allows more of the antibody to become associated with marker substance. Inasmuch as marker substance is itself associated with the lesion, antibody accumulation in, on, or near the lesion is increased. In this way, the lesion is more precisely located. The therapeutic agent's cytotoxic effects are also directed more precisely to the lesion and undesired cytotoxicity is minimized.

Instead of whole antibodies, antibody fragments specific for lesion-associated marker substances may be used in accordance with the present invention. Specifically, where it is sought to decrease liver and spleen uptake of antibody fragments and to increase the quantity of antibody fragments which complexes with marker substance, the human subject may be initially exposed to antibody fragments specific for the marker substance. At a time after this initial exposure such that the subject comes to recognize the antibody fragments, the same antibody fragment may be administered to the subject. The prior exposure to the antibody fragment decreases the uptake of the subequently administered antibody fragment by the subject's liver and spleen. As a corollary of this diminished uptake, more of the antibody fragments is retained by the blood and available for formation of complexes with the lesion-associated marker substance. Such fragments may be associated with detectable and/or therapeutically effective labels as previously described.

## Claims

1. A method for enhancing the biodistribution of antibody for localization in lesions comprising:
initially exposing a mammalian subject containing lesion-associated marker substance to antibody specific for said marker substance, and
after a period of time such that said marker substance has associated with said antibody, reexposing said mammal to a further amount of said antibody.

2. The method of claim 1 wherein said antibody is a monoclonal antibody of mammalian origin.

3. The method of claim 1 wherein said antibody is a fragment of a monoclonal antibody of mammalian origin.

4. The method of claim 1 wherein said exposure and reexposure to antibody is accomplished by intravenous, parenteral or subcutaneous injection or oral administration.

5. The method of claim 4 wherein said mammalian subject is a human.

6. The method of claim 5 wherein said further amount of said antibody is labeled with a detectable agent.

7. The method of claim 5 wherein said further amount of said antibody is labeled with a therapeutically effective agent.

8. The method of claim 5 wherein said further amount of said antibody is labeled with both a detectable agent and a therapeutically effective agent.

9. The method of claim 1 wherein said period of time such that said marker substance has associated with said antibody is from about one week to about three weeks.

10. The method of claim 6 or 8 wherein said detectable agent is a radionuclide suitable for gamma imaging or a magnetic contrast agent.

11. The method of claim 10 wherein said radionuclide is indium-111, technecium-99m, iodine-131, iodine-123, iodine-126, iodine-133, bromine-77, fluorine-18, indium 113m, gallium-67, gallium-68, ruthenium-95, ruthenium-97, ruthenium-103, ruthenium-105, mercury-197, mercury-203, scandium-47, tellurium-121m, tellurium-122m, tellurium-125m, rhenium-99m, rhenium-101, rhenium-105, thulium-165, thulium-167, thulium-168, thulium-202, or antimony.

12. The method of claim 10 wherein said magnetic contrast agent is gadolinium, manganese, or iron.

13. The method of claim 7 or 8 wherein said therapeutically effective agent is yttrium-90, methotrexate, copper-67, indium-194, cobalt, carbon-14, daunomycin, vincristine, 5-fluorouracil, BCNU, nitrogen mustard, vinblastin, cyclophosphamide, actinomycin D, 6-mercaptopurine, arabinosyl, cytosine, L-asparaginase, adriamycin, cytoxin, busulfan, chlorambucil, thiotepa, melphalan, thioguanine, hydroxyurea, procarbazine, o,p-D,D,D, FUDR, mithramycin, bleomycin, DTIC, CCNU, cis-platinum, or boron-10.

14. The method of claim 1 wherein said lesion-associated marker substance is oncofetal antigen, tumor antigen, a receptor, a hormone or an enzyme.

15. The method of claim 1 or 14 wherein said lesion-associated marker substance is carcinoembryonic antigen (CEA), human chorionic gonadotropin (HCG) and/or its beta subunit, alpha-fetoprotein (AFP), Ca 19-9, CA125, 132CS, tissue polypeptide antigen, calon-specific antigen-p (CSA$_p$), A 431, TA-4, prostatic acid phosphatase, pancreatic oncofetal antigen, T101, S 100, parathormane, B1.1, B72.3, placental alkaline phosphatase, calcitonin, mammary tumor-associated glycoproteins, galactyasyl transferase-II (gt-II), glial fibrillary acidic protein (GFA), tumor angiogenesis factor (TAF), prostatic acid phosphatase, pregnancy beta$_1$-globulin, beta$_2$-microglobulin ovarian cystadenocarcinoma-associated antigen (OCAA), ovarian tumor-specific antigen, common glioma antigen (CGA), cervical cancer antigens, glioembryonic antigen (GEA), common meningioma antigen (CMA), terminal deoxynucleotidyl transferase (TAT), and cytoplasmic melanoma-associated antigens.

16. The method of claim 5 wherein said initial exposure to antibody is carried out over a period of about four days.

17. A method for enhanced imaging of tumors in mammalian subjects near the liver and spleen comprising:

initially exposing said mammalian subject containing lesion-associated marker substance to antibody specific for said marker substance, and

after a period of time such that said marker substance has associated with said antibody, reexposing said mammal to a further amount of said antibody that is labeled with a detectable agent.

18. The method of claim 17 wherein said antibody is a monoclonal antibody of mammalian origin.

19. The method of claim 17 wherein said antibody is a fragment of a monoclonal antibody of mammalian origin.

20. The method of claim 17 wherein said exposure and reexposure to antibody is accomplished by intravenous, parenteral or subcutaneous injection or oral administration.

21. The method of claim 17 wherein said mammalian subject is a human.

22. The method of claim 17 wherein said further amount of antibody is labeled with a detectable agent.

23. The method of claim 17 wherein said period of time such that said marker substance has associated with said antibody is from about one week to about three weeks.

24. The method of claim 22 wherein said detectable agent is a radionuclide suitable for gamma imaging or a magnetic contrast agent.

25. The method of claim 24 wherein said radionuclide is indium-111, technecium-99m, iodine-131, iodine-123, iodine-126, iodine-133, bromine-77, fluorine-18, indium 113m, gallium-67, gallium-68, ruthenium-95, ruthenium-97, ruthenium-103, ruthenium-105, mercury-197, mercury-203, scandium-47, tellurium-121m, tellurium-122m, tellurium-125m, rhenium-99m, rhenium-101, rhenium-105, thulium-165, thulium-167, thulium-168, thallium-202, or antimony.

26. The method of claim 24 wherein said magnetic contrast agent is gadolinium, manganese, or iron.

27. The method of claim 17 wherein said lesion-associated marker substance is oncofetal antigen, tumor antigen, a receptor, a hormone or an enzyme.

28. The method of claim 17 or 27 wherein said lesion-associated marker substance is carcinoembryonic antigen (CEA), human chorionic gonadotropin (HCG) and/or its beta subunit, alpha-fetoprotein (AFP), Ca 19-9, CA125, 132CS, tissue polypeptide antigen, calon-specific antigen-p (CSA$_p$), A 431, TA-4, prostatic acid phosphatase, pancreatic oncofetal antigen, T101, S 100, parathormane, B1.1, B72.3, placental alkaline phosphatase, calcitonin, mammary tumor-associated glycoproteins, galactyasyl transferase-II (gt-II), glial fibrillary acidic protein (GFA), tumor angiogenesis factor (TAF), prostatic acid phosphatase, pregnancy beta$_1$-globulin, beta$_2$-microglobulin ovarian cystadenocarcinoma-associated antigen (OCAA), ovarian tumor-specific antigen, common glioma antigen (CGA), cervical cancer antigens, glioembryonic antigen (GEA), common meningioma antigen (CMA), terminal deoxynucleotidyl transferase (TAT), and cytoplasmic melanoma-associated antigens.

29. The method of claim 17 wherein said initial exposure to antibody is carried out over a period of about four days.

30. A method for enhanced therapy of lesions in mammalian subjects comprising:

initially exposing said mammalian subject containing lesion-associated marker substance to antibody specific for said marker substance, and

after a period of time such that said marker substance has associated with said antibody, reexposing said mammal to a further amount of said antibody that is labeled with a therapeutically effective agent.

31. The method of claim 30 wherein said antibody is a monoclonal antibody of mammalian origin.

32. The method of claim 30 wherein said antibody is a fragment of a monoclonal antibody of mammalian origin.

33. The method of claim 30 wherein said exposure and reexposure to antibody is accomplished by intravenous, parenteral or subcutaneous injection or oral administration.

34. The method of claim 30 wherein said mammalian subject is human.

35. The method of claim 30 wherein said further amount of antibody is labeled with a therapeutically effective agent.

36. The method of claim 30 wherein said period of time such that said marker substance has associated with said antibody is from about one week to about three weeks.

37. The method of claim 35 wherein said therapeutically effective agent is yttrium-90, methotrexate, copper-67, indium-194, cobalt, carbon-14, daunomycin, vincristine, 5-fluorouracil, BCNU, nitrogen mustard, vinblastin, cyclophosphamide, actinomycin D, 6-mercaptopurine, arabinosyl, cytosine, L-asparaginase, adriamycin, cytoxin, busulfan, chlorambucil, thiotepa, melphalan, thioguanine, hydroxyurea, procarbazine, o,p-D,D,D, FUDR, mikthramycin, bleomycin, DTIC, CCNU, cis-platinum, or boron-10.

38. The method of claim 30 wherein said lesion-associated marker substance is oncofetal antigen, tumor antigen, a receptor, a hormone or an enzyme.

39. The method of claim 30 or 38 wherein said lesion-associated marker substance is carcinoembryonic antigen (CEA), human chorionic gonadotropin (HCG) and/or its beta subunit, alpha-fetoprotein (AFP), Ca 19-9, CA125, 132CS, tissue polypeptide antigen, calon-specific antigen-p ($CSA_p$), A 431, TA-4, prostatic acid phosphatase, pancreatic oncofetal antigen, T101, S 100, parathormane, B1.1, B72.3, placental alkaline phosphatase, calcitonin, mammary tumor-associated glycoproteins, galactyasyl transferase-II (gt-II), glial fibrillary acidic protein (GFA), tumor angiogenesis factor (TAF), prostatic acid phosphatase, pregnancy $beta_1$-globulin, $beta_2$-microglobulin ovarian cystadenocarcinoma-associated antigen (OCAA), ovarian tumor-specific antigen, common glioma antigen (CGA), cervical cancer antigens, glioembryonic antigen (GEA), common meningioma antigen (CMA), terminal deoxynucleotidyl transferase (TAT), and cytoplasmic melanoma-associated antigens.

40. The method of claim 30 wherein said initial exposure to antibody is carried out over a period of about four days.